# EUROPEAN PATENT APPLICATION

(11) **EP 4 483 827 A1**
(43) Date of publication of application: **01.01.2025**
(21) Application number: 23766346.3
(22) Date of filing: 25.01.2023
(51) Int. Cl.: A61B 18/14

(54) **HIGH FREQUENCY ELECTRODE NEEDLE WITH TEMPERATURE-MEASUREMENT FUNCTION**

(30) Priority: 10.03.2022 JP 2022037291
(71) Applicant: Kabushiki Kaisha Top, Adachi-ku, Tokyo 120-0035 (JP)
(72) Inventor: MURATA, Koji, Tokyo 120-0035 (JP); MIYAZAWA, Yoshihiro, Tokyo 120-0035 (JP)
(74) Representative: Schön, Christoph
(86) International application number: PCT/JP2023/002181
(87) International publication number: WO 2023/171166

(57) **Abstract**

Provided is a high-frequency electrode needle having a temperature measurement function, which can improve a temperature measurement accuracy at a needle tip while avoiding a decrease in function. A temperature measurement contact C1 (temperature measurement portion) of a thermocouple (14) is disposed while being positioned at a front side of a rear end portion P2 of an inclined cutting edge surface P and a rear side of the inclined cutting edge surface P in a lumen (for example, internal space of a needle tube (11)) of the needle tube (11). That is, a temperature measurement contact C1 is disposed at a position where a cross-section perpendicular to an axial direction of the needle tube (11) partially surrounds the internal space.

## Description

### Technical Field

The present invention relates to a high-frequency electrode needle having a temperature measurement function, which can cauterize a specific tissue by discharging a high-frequency current and/or performing a nerve block by a pulse high frequency and/or administration of anesthetic solution from a needle tip portion.

### Background Art

A balloon catheter configured to prevent disconnection of a wire connected to a temperature sensor has been proposed (for example, see Patent Literature 1). Specifically, a copper wire for supplying a high-frequency voltage of a high-frequency generator to an electrode for high-frequency heating is connected to one end of a thermocouple sensor. A coil that separates the high-frequency signal is connected to the other end of the thermocouple sensor. The coil is incorporated in a catheter body. The copper wire and the coil are electrically connected to a thermometer.

With this configuration, the copper wire for the electrode is used as one wire of the thermocouple sensor, and a high frequency from a high-frequency generator is blocked by the coil connected to the other end of the thermocouple sensor, and a direct current temperature signal of the thermocouple sensor is output via the copper wire and the coil.

### Citation List

### Patent Literature

Patent Literature 1: Japanese Patent Application Laid-Open No. H05-293183

### Summary of Invention

### Technical Problem

However, in the treatment using the high-frequency electrode needle having a temperature measurement function, it is preferable to measure a temperature of a needle tip that comes into contact with or is close to an affected part.

Therefore, an object of the present invention is to provide a high-frequency electrode needle having a temperature measurement function, which can improve a temperature measurement accuracy at a needle tip while avoiding a decrease in function.

### Solution to Problem

The present invention relates to a high-frequency electrode needle having a temperature measurement function (hereinafter, referred to as a "high-frequency electrode needle" for simplification of expression), which includes: a tubular needle tube having an inclined cutting edge surface formed at a needle tip; a tubular needle base to which the needle tube is attached at a tip end portion; a thermocouple including a pair of thermocouple wires electrically connected at a temperature measurement contact that is disposed at the tip end portion of the needle tube; and a high-frequency wire disposed along an outside of the needle base and electrically connected to the needle tube to form a high-frequency contact.

In the high-frequency electrode needle according to a first aspect of the present invention, the temperature measurement contact is disposed at a rear side of the inclined cutting edge surface while being positioned in the lumen of the needle tube.

With the high-frequency electrode needle of the configuration, the temperature measurement contact (temperature measurement portion) is disposed while being positioned at the rear side of the inclined cutting edge surface in the lumen of the needle tube. Therefore, the decrease in a puncture function of the needle tube can be avoided as compared with a case in which the temperature measurement contact of the thermocouple is disposed on the outside of the needle tube and/or at a front side of the inclined cutting edge surface. Further, the measurement accuracy of a temperature of the needle tube at a position close to the inclined cutting edge surface of the needle tube is improved.

In the high-frequency electrode needle having a temperature measurement function of the configuration, it is preferable that the temperature measurement contact is disposed while being positioned at a front side of a rear end portion of the inclined cutting edge surface and at the rear side of the inclined cutting edge surface in the lumen of the needle tube.

With the high-frequency electrode needle of the configuration, the temperature measurement contact (temperature measurement portion) is disposed while being positioned at the front side of the rear end portion of the inclined cutting edge surface and the rear side of the inclined cutting edge surface in the lumen of the needle tube. That is, the temperature measurement contact is disposed at the position where a cross-section perpendicular to an axial direction of the needle tube partially surrounds the internal space. Therefore, the decrease in a puncture function of the needle tube can be avoided as compared with a case in which the temperature measurement contact of the thermocouple is disposed on the outside of the needle tube and/or at a front side of the inclined cutting edge surface. In addition, as compared with a case in which the temperature measurement contact is disposed at the rear side from the rear end portion of the inclined cutting edge surface in the lumen of the needle tube, an effective area of the lumen of the needle tube is ensured to be large, and in a case in which the lumen constitutes a chemical liquid passage, a decrease in a chemical liquid administration function of the needle tube can be avoided. Further, the measurement accuracy of a temperature of the needle tube at a position close to the inclined cutting edge surface of the needle tube is improved.

With the high-frequency electrode needle having a temperature measurement function of the configuration, it is preferable that each of the pair of thermocouple wires is fixed to the needle base while being interposed between an inner surface of the needle base and an outer surface of a chemical liquid tube that is inserted into the inside of the needle base and liquid-tightly bonded to the inside of the needle base, so that the temperature measurement contact is disposed while being positioned in the lumen of the needle tube.

With the high-frequency electrode needle of the configuration, the pair of thermocouple wires are fixed to the needle base while being interposed between the inner surface of the needle base and the outer surface of the chemical liquid tube. Therefore, in order to position the temperature measurement contact with respect to the needle tube, the temperature measurement contact need not be adhered to the needle tube by an adhesive, and thus the effective area of the lumen of the needle tube is reduced by the adhesive, and when the lumen constitutes the chemical liquid passage, the decrease in the chemical liquid administration function of the needle tube can be avoided.

In the high-frequency electrode needle of the configuration, it is preferable that a thermocouple channel is formed through a side wall of the needle tube in a longitudinal direction, and each of the pair of thermocouple wires is disposed in an internal space of the thermocouple channel, and the temperature measurement contact is disposed while being positioned in the internal space of the thermocouple channel, which serves as the lumen of the needle tube.

With the high-frequency electrode needle of the configuration, the pair of thermocouple wires and the temperature measurement contact are disposed in the thermocouple channel formed on the side wall that surrounds the internal space of the needle tube (partially surrounds the thermocouple channel in a region of the inclined cutting edge surface). Therefore, as compared with a case in which the thermocouple is disposed in the internal space of the needle tube, a reduction in the effective area of the internal space and a decrease in the chemical liquid administration function of the needle tube through the internal space can be avoided.

In high-frequency electrode needle of the configuration, it is preferable that the needle tube includes a first needle tube having the inclined cutting edge surface formed at the needle tip, and a second needle tube connected to a rear end portion of the first needle tube, the first needle tube has a first thermocouple channel formed through a side wall of the first needle tube in the longitudinal direction, the second needle tube has a high-frequency channel formed through a side wall of the second needle tube in the longitudinal direction, and a second thermocouple channel formed through the side wall of the second needle tube in the longitudinal direction and configured to communicate with the first thermocouple channel, the high-frequency wire is inserted into the high-frequency channel from a rear end portion of the high-frequency wire, and is electrically connected to the rear end portion of the first needle tube to form a high-frequency contact, and each of the pair of thermocouple wires is disposed in the internal space of the thermocouple channel which includes the first thermocouple channel and the second thermocouple channel communicating with each other, and the temperature measurement contact is disposed while being positioned in an internal space of the first thermocouple channel, which serves as the lumen of the needle tube.

With the high-frequency electrode needle of the configuration, the needle tube includes the first needle tube and the second needle tube connected to each other. The first thermocouple channel that extends in the longitudinal direction is formed on the side wall that surrounds an internal space of the first needle tube (partially surrounds the internal space in the region of the inclined cutting edge surface). The second thermocouple channel that extends in the longitudinal direction is formed on a side wall that surrounds the internal space of the second needle tube. The thermocouple channel formed in the side wall of the needle tube includes the first and second thermocouple channels that communicate with each other. The pair of thermocouple wires and the temperature measurement contact are disposed in the thermocouple channel. Therefore, as compared with a case in which the thermocouple is disposed in the internal space of the needle tube, a reduction in the effective area of the internal space and a decrease in the chemical liquid administration function of the needle tube through the internal space can be avoided. In addition, since the high-frequency wire is disposed in the high-frequency channel, a decrease in the puncture function of the needle tube can be avoided.

In the high-frequency electrode needle of the configuration, it is preferable that the temperature measurement contact is formed by electrically connecting one of the pair of thermocouple wires to the needle tube serving as the other of the pair of thermocouple wires.

With the high-frequency electrode needle of the configuration, the number of thermocouple wires disposed in the lumen of the needle tube can be set to one (single wire), so that the effective area in the lumen can be decreased, and furthermore, when the chemical liquid passage is formed in the lumen, a decrease in the chemical liquid administration function of the needle tube can be avoided.

In the high-frequency electrode needle of the configuration, it is preferable that an insulating tube is disposed in the lumen of the needle tube while being positioned with respect to the needle tube, and each of the pair of thermocouple wires is disposed in an internal space of the insulating tube, and the temperature measurement contact is disposed while being positioned in the lumen of the needle tube and the internal space of the insulating tube.

With the high-frequency electrode needle of the configuration, since the thermocouple is disposed in the internal space of the needle tube, a decrease in the puncture function of the needle tube can be avoided as compared with a case where the thermocouple is disposed on the outside of the needle tube.

In the high-frequency electrode needle of the configuration, it is preferable that the needle tube is covered with an insulating member except for a designated region.

With the high-frequency electrode needle of the configuration, the high-frequency discharge is efficiently performed from the designated region exposed without being covered with the insulating member to a treatment target site in the needle tube that is present in the patient's body.

In a high-frequency electrode needle according to a second aspect of the present invention, in a state in which a gap between an outer surface of the needle tube and an inner surface of the insulating tube extends in a longitudinal direction of the needle tube, and in a state in which the outer surface of the needle tube and the inner surface of the insulating tube are bonded at a position except for the gap, at least a part of the outer surface of the needle tube is covered with the insulating tube, and the pair of thermocouple wires are inserted into the gap from a rear end portion of the pair of thermocouple wires, and the temperature measurement contact is disposed while being positioned at a rear side of the inclined cutting edge surface in an internal space of the insulating tube.

With the high-frequency electrode needle of the configuration, the temperature measurement contact (temperature measurement portion) of the thermocouple is disposed while being positioned at a rear side of the inclined cutting edge surface in the gap extending in the longitudinal direction between the needle tube and the insulating tube surrounding the needle tube. Therefore, the decrease in a puncture function of the insulating tube can be avoided as compared with a case in which the temperature measurement contact of the thermocouple is disposed on the outside of the needle tube and/or at a front side of the inclined cutting edge surface. In addition, as compared with a case in which the temperature measurement contact is disposed in the lumen of the needle tube, an effective area of the lumen of the needle tube is ensured to be large, and in a case in which the lumen constitutes a chemical liquid passage, a decrease in a chemical liquid administration function of the needle tube can be avoided. Further, the measurement accuracy of a temperature of the needle tube at a position close to the inclined cutting edge surface of the needle tube is improved.

### Brief Description of Drawings

FIG. 1 is a configuration explanatory view of a high-frequency electrode needle as a first embodiment of the present invention.
FIG. 2 is a detailed configuration explanatory view of an X1 part (tip end portion of a needle tube) of FIG. 1.
FIG. 3 is a detailed configuration explanatory view of the X1 part (tip end portion of the needle tube) of FIG. 1.
FIG. 4 is a detailed configuration explanatory view of an X2 part (needle base and winding portion) of FIG. 1.
FIG. 5A is another configuration explanatory view of the needle base and the winding portion.
FIG. 5B is another configuration explanatory view of the needle base and the winding portion.
FIG. 6 is an explanatory view related to an arrangement aspect of a high-frequency wire in the needle base.
FIG. 7 is a detailed configuration explanatory view of an X3 part (thermocouple connector) of FIG. 1.
FIG. 8 is a configuration explanatory view of a needle tube of a high-frequency electrode needle according to a second embodiment of the present invention.
FIG. 9 is a configuration explanatory view of a needle tube of a high-frequency electrode needle as a third embodiment of the present invention.
FIG. 10 is a configuration explanatory view of a needle tube of a high-frequency electrode needle according to a fourth embodiment of the present invention.
FIG. 11 is a configuration explanatory view of a needle tube of a high-frequency electrode needle according to a fifth embodiment of the present invention.
FIG. 12 is a configuration explanatory view of a needle tube of a high-frequency electrode needle according to a sixth embodiment of the present invention.

### Description of Embodiments

### (First Embodiment)

### (Configuration of High-Frequency Electrode Needle)

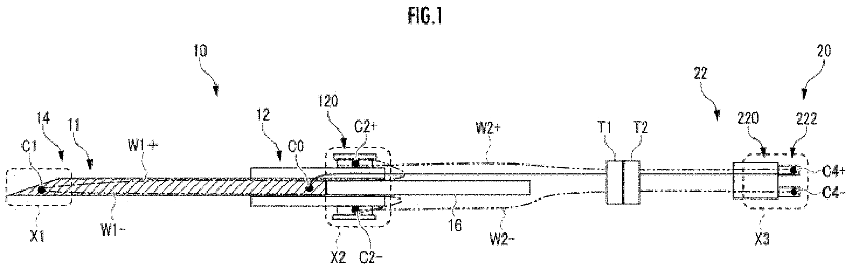

A high-frequency electrode needle as a first embodiment of the present invention, which is schematically illustrated in FIG. 1, includes a first unit 10 and a second unit 20.

As illustrated in FIG. 1, the first unit 10 includes a substantially cylindrical needle tube 11, a substantially cylindrical or substantially truncated conical cylindrical needle base 12 to which the needle tube 11 is attached at a tip end portion, a high-frequency wire W0 (see the solid line) electrically connected to the needle tube 11, a thermocouple 14 including a pair of thermocouple wires W1+ and W1- (see the one-dot chain line), a pair of compensation wires W2+ and W2- (see the two-dot chain line), and a first connector T1.

The needle tube 11 is attached to the needle base 12 in a state in which a rear end portion thereof is inserted from the tip end portion to an inside or hollow space of the needle base 12 and the inside of the needle tube 11 and the inside of the needle base 12 communicate with each other. A tip end portion of a chemical liquid tube 16 is inserted into the inside of the needle base 12 from the rear end portion. Accordingly, a chemical liquid is supplied from a chemical liquid supply device connected to the rear end portion of the chemical liquid tube 16 to the inside of the needle tube 11 through the chemical liquid tube 16, and the chemical liquid is discharged from the tip end portion of the needle tube 11.

The pair of thermocouple wires W1+ and W1- are electrically connected to each other at a temperature measurement contact C1 disposed at the tip end portion of the needle tube 11. The pair of thermocouple wires W1+ and W1- and the pair of compensation wires W2+ and W2- are electrically connected to each other at a pair of compensation contacts C2+ and C2-, respectively. As will be described below, a pair of winding portions 120 that locally protrude to the outside of the needle base 12 include the pair of compensation contacts C2+ and C2-. The high-frequency wire W0 and the pair of compensation wires W2+ and W2- may be collectively accommodated in a wiring tube (not illustrated) having insulating properties and flexibility. In this case, the wire may be led from a tip end portion of the wiring tube to the rear end portion of the needle base 12.

The needle tube 11 is formed of, for example, a conductive material such as stainless steel. The needle base 12 is formed of, for example, an insulating material such as an epoxy resin. The high-frequency wire W0 is formed of, for example, a copper wire (for example, ϕ0.18 mm) or an enameled wire. One thermocouple wire W1+ and one compensation wire W2+ are formed of, for example, a chromel wire (for example, ϕ0.1 mm). The other thermocouple wire W1- and the other compensation wire W2- are formed of, for example, an alumel wire (for example, ϕ0.1 mm). One compensation wire W2+ may be formed of a copper wire (for example, ϕ0.1 mm), and the other compensation wire W2- may be formed of a constantan wire (for example, ϕ0.1 mm). One thermocouple wire W1+ may be formed of a copper wire, and the other thermocouple wire W1- may be formed of a constantan wire. The high-frequency wire W0, the thermocouple wires W1+ and W1-, and/or the compensation wires W2+ and W2- may be covered with an insulating coating layer, except for a part constituting an electrical contact.

As illustrated in FIG. 1, the second unit 20 includes a high-frequency wire W0 (see the solid line), a pair of compensation wires W2+ and W2- (see the two-dot chain line), a thermocouple connector 22, and a second connector T2. The thermocouple connector 22 includes a body 220 and a pair of terminals 222 that protrude from the body 220. The pair of terminals 222 are inserted into a terminal receiving portion having a high-frequency power supply device (not illustrated) having a temperature measurement function to constitute reference temperature contacts C4+ and C4- electrically connected to the device (particularly, a temperature measurement device).

The first unit 10 and the second unit 20 are connected to each other in a removable manner via the first connector T1 and the second connector T2. The first connector T1 and the second connector T2 are connected to each other, so that the high-frequency wire W0 constituting the first unit 10, each of the pair of the compensation wires W2+ and W2- and the high-frequency wire W0 constituting the second unit 20, and each of the pair of the compensation wires W2+ and W2- are electrically connected to each other.

### (Configuration of Needle Tube)

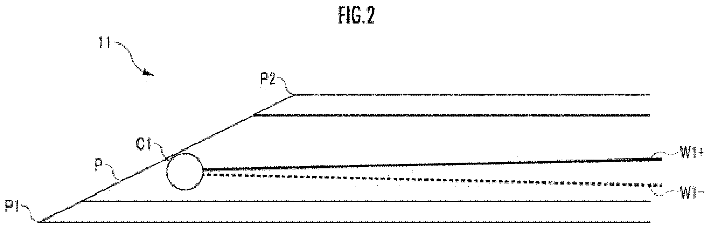

As illustrated in FIG. 2, the tip end portion of the needle tube 11 is formed with an inclined cutting edge surface P having a substantially planar shape and inclined with respect to a central axis of the needle tube 11. As illustrated in FIG. 2, the temperature measurement contact C1, at which the pair of thermocouple wires W1+ and W1-constituting the thermocouple 14 are electrically connected to each other, is disposed inside the needle tube 11 at a rear side of the inclined cutting edge surface P and midway between a tip end portion P1 and a rear end portion P2 of the inclined cutting edge surface P. The temperature measurement contact C1 may be disposed at a position corresponding to the rear end portion P2 of the inclined cutting edge surface P or the rear side of the rear end portion P2 of the inclined cutting edge surface P (for example, rear side by a distance equal to or less than 0.2 times a length of the inclined cutting edge surface P in an axial direction of the needle tube 11). The same applies to other embodiments.

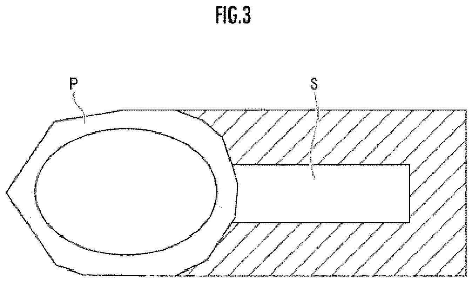

As illustrated in FIG. 3, the needle tube 11 is covered with an insulating material (for example, a fluorine coating material) except for the inclined cutting edge surface P and a local region S on an outer surface of the tip end portion. The local region S is a substantially rectangular region when viewed from a top, which extends in a range corresponding to the rear end portion P2 of the inclined cutting edge surface P in a circumferential direction of the needle tube 11 on the outer surface of the tip end portion, and extends in the longitudinal direction (a direction parallel to the central axis) of the needle tube 11. The shape of the region may be variously changed, in addition to the substantially rectangular shape, such as a substantially triangular shape, a substantially trapezoidal shape, a substantially circular shape, a substantially oval shape, a substantially elliptical shape, or a combination of these shapes.

In another embodiment, the tip end portion of the needle tube 11 may be covered with an insulating material except for at least a part of the inclined cutting edge surface P, or the tip end portion of the needle tube 11 may be covered with an insulating material except for the entire circumference of the inclined cutting edge surface P and the outer surface of the tip end portion of the needle tube 11.

### (Configuration of Needle Base)

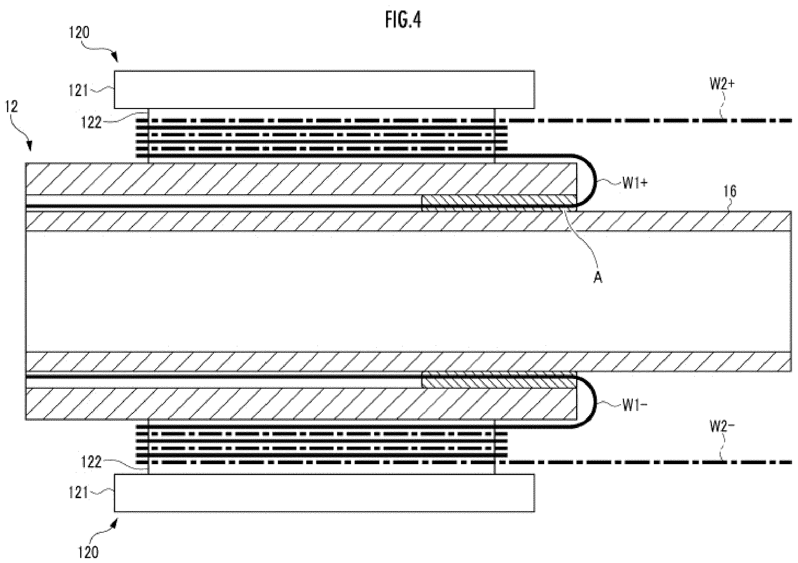

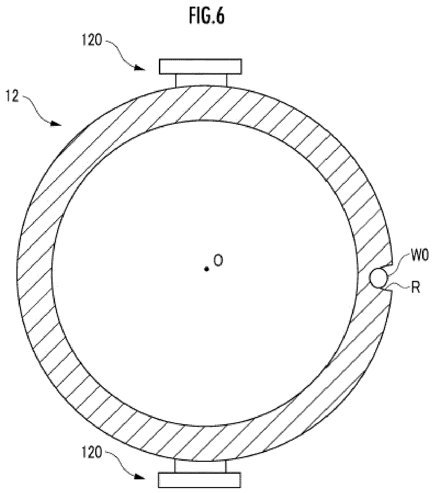

As illustrated in FIG. 4, the pair of winding portions 120 that locally protrude outward are formed on the needle base 12. As illustrated in FIG. 6, the pair of winding portions 120 are disposed to have two-time rotational symmetry with respect to a central axis O of the needle base 12. The pair of winding portions 120 may be disposed such that an azimuthal angle deviation with respect to the central axis O of the needle base 12 is not 180°, but is another value such as 45°, 60°, 75°, 90°, 120°, 135°, or 150°. The winding portion 120 is formed such that a proximal portion 122 is recessed or narrowed over the entire circumference with respect to the distal portion 121 of the needle base 12 about the central axis. For example, the proximal portion 122 may be formed in a substantially elongated cylindrical shape extending along the central axis of the needle base 12, and the distal portion 121 may be formed in a substantially elongated disc shape or a substantially elongated cylindrical shape that protrudes over the entire circumference of the proximal portion 122.

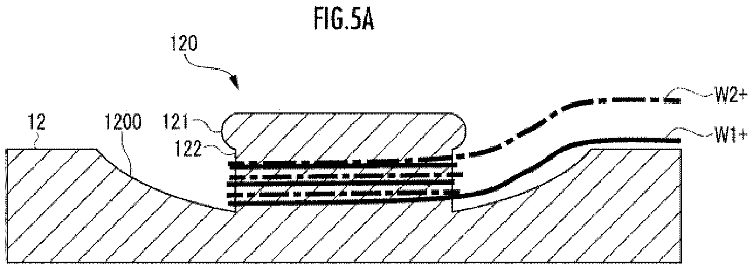

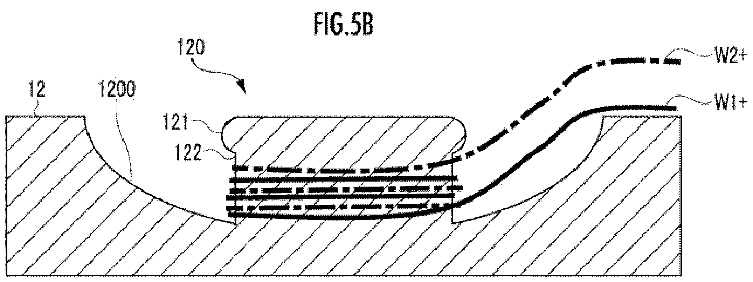

In the present embodiment, the winding portion 120 protrudes radially outward from the substantially cylindrical or substantially truncated conical cylindrical outer surface of the needle base 12. However, in another embodiment, as illustrated in FIG. 5A, a recessed portion 1200 that is locally recessed may be formed in the needle base 12 from the substantially cylindrical or substantially truncated conical cylindrical outer surface of the needle base 12, and the winding portion 120 may be configured to protrude radially outward from a bottom surface of the recessed portion 1200. In this case, the winding portion 120 may partially and radially protrude outside of the outer surface of the needle base 12 from the recessed portion of the needle base 12. In addition, as illustrated in FIG. 5B, the winding portion 120 may be entirely accommodated in the recessed portion of the needle base 12, for example, a tip end surface or a top surface of the winding portion 120 is disposed on the substantially same curved surface as the outer surface of the needle base 12.

As illustrated in FIG. 6, a groove R is formed in the outside of the needle base 12 along the longitudinal direction, and the high-frequency wire W0 is disposed along the groove R. For example, the high-frequency wire W0 is continuously provided on the inside of the needle base 12 through a slit formed in a side wall of the needle base 12 so as to extend from the tip end portion in the axial direction, and is electrically connected to the rear end portion of the needle tube 11 at a high-frequency contact C0 illustrated in FIG. 1 by soldering or the like.

In the present embodiment, an azimuthal angle of the groove R with respect to the central axis O of the needle base 12 is shifted by approximately 90° from an azimuthal angle of each of the pair of winding portions 120. The azimuthal angle of the groove R with respect to the central axis O of the needle base 12 may be optionally changed, for example, to 30°, 45°, 60°, or the like with respect to the azimuthal angle of one winding portion 120, within a range in which the wire wound around the one winding portion 120 and the high-frequency wire W0 disposed in the groove R do not interfere with each other or are not electrically conducted.

As illustrated in FIG. 4, one compensation contact C2+ is formed by winding one thermocouple wire W1+ and one compensation wire W2+ around one winding portion 120 in the proximal portion 122. As illustrated in FIG. 4, the other compensation contact C2-is formed by winding the other thermocouple wire W1- and the other compensation wire W2- around the other winding portion 120 in an overlapped manner at the proximal portion 122. The number of turns of each wire is, for example, "4", but may be the same or different from each other, and may be a number of turns other than "2" or "8" or the like.

It is preferable that the thermocouple wires W1+ and W1- and/or the compensation wires W2+ and W2- are wound around the winding portion 120 such that free end surfaces of the thermocouple wires W1+ and W 1- and/or the compensation wires W2+ and W2- are directed to a side opposite to the needle tube.

As illustrated in FIG. 4, each of the pair of thermocouple wires W1+ and W1- is wound around the winding portion 120 in this manner, and then wraps around from the rear end portion of the needle base 12 to be continuously provided to the inside of the needle base 12. As schematically shown in FIG. 4, each of the pair of thermocouple wires W1+ and W1-, which wraps around from the rear end portion of the needle base 12 to be continuously provided to the inside of the needle base 12, is fixed to the needle base 12 while being interposed between the inner surface of the needle base 12 and the outer surface of the chemical liquid tube 16. The needle base 12 and the chemical liquid tube 16 are liquid-tightly fixed by an adhesive layer A over the entire circumference. In FIG. 4, for easy understanding of the configuration, cross-sections of the needle base 12, the chemical liquid tube 16, and the adhesive layer A are illustrated.

A tubular needle base cover, which is guided from a side of the needle tube 11, may be attached to the outside of the needle base 12. The needle base 12 and the needle base cover attached to the needle base 12 may be fixed to each other by the adhesive.

### (Configuration of Thermocouple Connector)

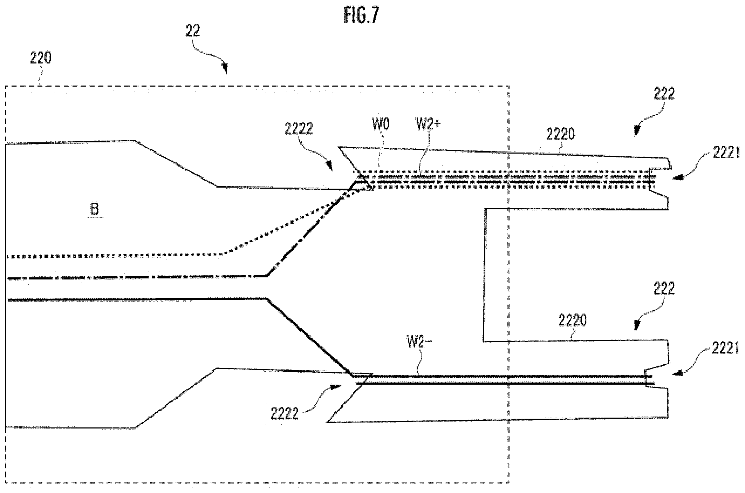

As schematically illustrated in FIG. 7, the thermocouple connector 22 includes a body 220 (housing) and a pair of terminals 222 that protrude from the body 220. The terminal 222 includes a pair of insulating base bodies 2220 which are spaced apart from each other in a lateral direction of a substantially rectangular substrate B, which is formed of an insulating material, from a tip end portion of the substrate B, and which protrude approximately parallel to the longitudinal direction of the substrate B. In the insulating base body 2220, a tip end recessed portion 2221, which is recessed in a substantially tongue piece shape in the longitudinal direction, is formed in a tip end portion that protrudes from the body 220. In the insulating base body 2220, a rear end recessed portion 2222, which is recessed in a substantially undercut shape in the longitudinal direction, is formed in the rear end portion accommodated in the body 220.

One terminal 222 provided on the upper side of FIG. 7 includes one insulating base body 2220, and one compensation wire W2+ and the high-frequency wire W0 (see dotted lines) that are wound around the one insulating base body 2220 so as not to overlap each other via the tip end recessed portion 2221 and the rear end recessed portion 2222. As illustrated in FIG. 7, the one compensation wire W2+ wound around the one insulating base body 2220 is interposed between the high-frequency wires W0 wound around the one insulating base body 2220 with a space therebetween.

The other terminal 222 on the lower side of FIG. 7 includes the other insulating base body 2220 and the other compensation wire W2- that is wound around the other insulating base body 2220 via the tip end recessed portion 2221 and the rear end recessed portion 2222.

### (Other Embodiments of Present Invention)

In the above embodiment, the first unit 10 and the second unit 20 are connected to each other in a removable manner via the first connector T1 and the second connector T2, but in other embodiments, the first unit 10 and the second unit 20 may be connected to each other in a non-removable manner via the first connector T1 and the second connector T2, or wires constituting each of the first unit 10 and the second unit 20 may be continuously provided.

The high-frequency electrode needle may include only the first unit 10. In this case, the first connector T1 may be omitted.

### (Second Embodiment)

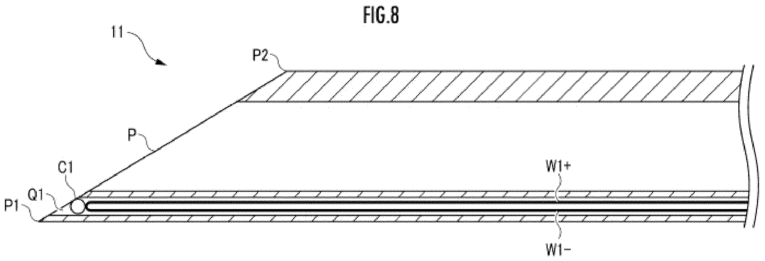

As illustrated in FIG. 8, the needle tube 11 constituting the high-frequency electrode needle as a second embodiment of the present invention is formed with a thermocouple channel Q1 formed through the side wall portion of the needle tube 11 in a direction substantially parallel to the central axis at an azimuthal angle position corresponding to the tip end portion P1 of the inclined cutting edge surface P about the central axis.

The pair of thermocouple wires W1+ and W1- are inserted into the thermocouple channel Q1 from the rear end portion thereof, and the temperature measurement contact C1 is disposed on the inside of the thermocouple channel Q1 at the rear side of the inclined cutting edge surface P and midway between the tip end portion P1 and the rear end portion P2 of the inclined cutting edge surface P. Since the configuration of the high-frequency electrode needle of the second embodiment other than the above-described configuration is the same as that of the first embodiment, the description thereof will be omitted.

### (Third Embodiment)

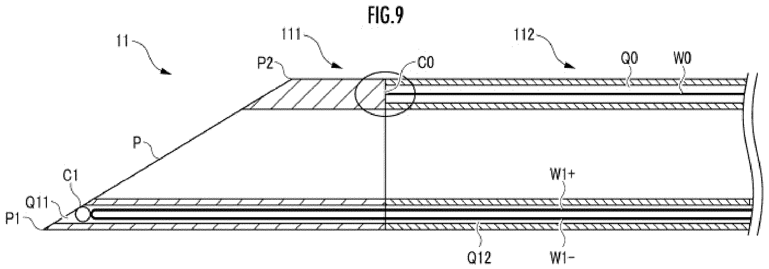

As illustrated in FIG. 9, the needle tube 11 constituting the high-frequency electrode needle according to a third embodiment of the present invention includes a first needle tube 111 and a second needle tube 112. The first needle tube 111 is formed of a substantially cylindrical conductive member of which a tip end surface is the inclined cutting edge surface P and a rear end surface is a substantially flat surface perpendicular to the central axis. The second needle tube 112 is formed of a substantially cylindrical insulating member. The rear end surface of the first needle tube 111 and the tip end surface of the second needle tube 112 are adhered or bonded to each other.

The first needle tube 111 is formed with a first thermocouple channel Q11 which is formed through a side wall portion of the first needle tube 111 in a direction substantially parallel to the central axis at an azimuthal angle position corresponding to the tip end portion P1 of the inclined cutting edge surface P about the central axis. The second needle tube 112 is formed with a second thermocouple channel Q12 which is formed through a side wall portion of the second needle tube 112 in the direction substantially parallel to the central axis at the azimuthal angle position corresponding to the tip end portion P1 of the inclined cutting edge surface P about the central axis. The first thermocouple channel Q11 and the second thermocouple channel Q12 communicate with each other to constitute one thermocouple channel. The second needle tube 112 is formed with a high-frequency channel Q0 which is formed through the side wall portion of the second needle tube 112 in the direction substantially parallel to the central axis at the azimuthal angle position corresponding to the rear end portion P2 of the inclined cutting edge surface P about the central axis.

The pair of thermocouple wires W1+ and W1- are inserted into one thermocouple channel including the first thermocouple channel Q11 and the second thermocouple channel Q12 from the rear end portion thereof, and the temperature measurement contact C1 is disposed on the inside of the first needle tube 111 at the rear side of the inclined cutting edge surface P and midway between the tip end portion P1 and the rear end portion P2 of the inclined cutting edge surface P. The high-frequency wire W0 is inserted into the high-frequency channel Q0 from the rear end portion thereof, and the tip end portion of the high-frequency wire W0 is electrically connected to the rear end surface of the first needle tube 111 by soldering or the like, thereby constituting the high-frequency contact C0. Since the configuration of the high-frequency electrode needle of the third embodiment other than the above-described configuration is the same as that of the first embodiment, the description thereof will be omitted.

### (Fourth Embodiment)

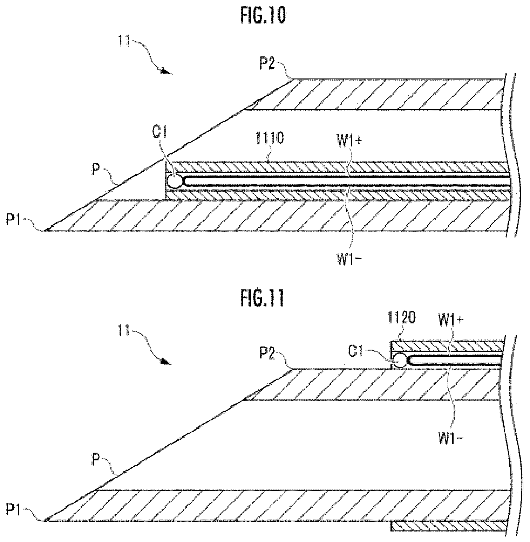

As illustrated in FIG. 10, the insulating tube 1110, which extends to the inside of the needle tube 11 in a direction substantially parallel to the central axis, is fixed to the needle tube 11 constituting the high-frequency electrode needle as a fourth embodiment of the present invention, at an azimuthal angle position corresponding to the tip end portion P1 of the inclined cutting edge surface P about the central axis. The outer surface of the insulating tube 1110 may be adhered to the inner surface of the needle tube 11, and as the insulating tube 1110 is adhered or mechanically fixed to the needle base 12, the insulating tube 1110 may be disposed while being positioned with respect to the needle tube 11. The pair of thermocouple wires W1+ and W1- are disposed in an internal space of the insulating tube 1110, and the temperature measurement contact C1 is disposed in the internal space of the insulating tube 1110 at the rear side of the inclined cutting edge surface P and midway between the tip end portion P1 and the rear end portion P2 of the inclined cutting edge surface P. Since the configuration of the high-frequency electrode needle of the fourth embodiment other than the above-described configuration is the same as that of the first embodiment, the description thereof will be omitted.

### (Fifth Embodiment)

As illustrated in FIG. 11, the needle tube 11 constituting the high-frequency electrode needle according to a fifth embodiment of the present invention surrounds over the entire circumference except for the tip end portion of the insulating tube 1120. The outer surface of the needle tube 11 and the inner surface of the insulating tube 1120 are adhered or bonded to each other at a position except for a gap therebetween extending in the longitudinal direction. The gap extends in a direction substantially parallel to the central axis or in the longitudinal direction at an azimuthal angle position corresponding to the rear end portion P2 of the inclined cutting edge surface P about the central axis of the needle tube 11. A part of the inner surface of the insulating tube 1120 may be adhered to a part of the outer surface of the needle tube 11, and as the insulating tube 1120 is adhered or mechanically fixed to the needle base 12, the insulating tube 1120 may be disposed while being positioned with respect to the needle tube 11.

The insulating tube 1120 may include an insulating member that covers the needle tube 11 except for a designated region, and the shape, the size, and the arrangement aspect of the designated region may be changed in various ways by changing the shape of the tip end portion of the insulating tube 1120 in various ways.

The pair of thermocouple wires W1+ and W1- are inserted into a gap between the needle tube 11 and the insulating tube 1120 from the rear end portion thereof, and the temperature measurement contact C1 is disposed in the internal space of the insulating tube 1120 at the rear side of the inclined cutting edge surface P and the rear side of the rear end portion P2 of the inclined cutting edge surface P. Since the configuration of the high-frequency electrode needle of the fifth embodiment other than the above-described configuration is the same as that of the first embodiment, the description thereof will be omitted.

### (Sixth Embodiment]

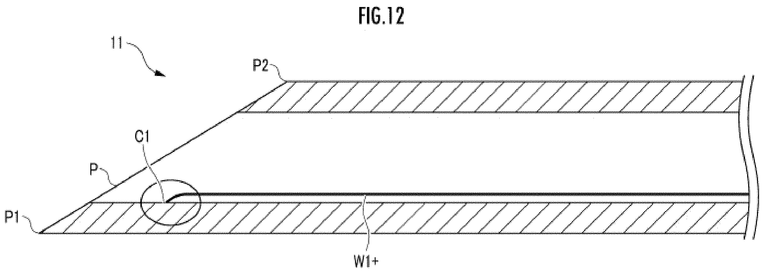

As illustrated in FIG. 12, the needle tube 11, which constitutes the high-frequency electrode needle as a sixth embodiment of the present invention, is formed with the temperature measurement contact C1 by electrically bonding, through soldering, welding, or the like, a thermocouple wire of one pole (W1+ in FIG. 12, but may be W1-) inserted from the rear end portion of the needle tube 11 through the inside, at an azimuthal angle position corresponding to the tip end portion P1 of the inclined cutting edge surface P about the central axis. That is, in the sixth embodiment, the needle tube 11 constitutes the other thermocouple wire, and the needle tube 11 is electrically connected to one compensation wire. The temperature measurement contact C1 is disposed on the inside of a side wall of the needle tube 11 at the rear side of the inclined cutting edge surface P and midway between the tip end portion P1 and the rear end portion P2 of the inclined cutting edge surface P. Since the configuration of the high-frequency electrode needle of the sixth embodiment other than the above-described configuration is the same as that of the first embodiment, the description thereof will be omitted.

The temperature measurement contact C1 in the high-frequency electrode needles of the first embodiment, the second embodiment, and the third embodiment may be formed in the same manner as in the sixth embodiment. In this case, in the high-frequency electrode needle of the third embodiment illustrated in FIG. 9, the thermocouple wire or the compensation wire may be electrically connected to the first needle tube 111.

### Advantageous Effect of Invention

With the high-frequency electrode needle of the configuration, the temperature measurement contact C1 (temperature measurement portion) of the thermocouple 14 is disposed while being positioned at the front side of the rear end portion P2 of the inclined cutting edge surface P and the rear side of the inclined cutting edge surface P in the lumen (for example, internal space of a needle tube 11) of the needle tube 11 (see FIG. 2). That is, a temperature measurement contact C1 is disposed at a position where a cross-section perpendicular to an axial direction of the needle tube 11 partially surrounds the internal space. Therefore, the decrease in the puncture function of the needle tube 11 can be avoided as compared with a case in which the temperature measurement contact C1 of the thermocouple 14 is disposed on the outside of the needle tube 11 and/or at the front side of the inclined cutting edge surface P. In addition, as compared with a case in which the temperature measurement contact C1 is disposed at the rear side from the rear end portion P2 of the inclined cutting edge surface P in the lumen of the needle tube 11, the effective area of the lumen of the needle tube 11 is ensured to be large, and in a case in which the lumen constitutes the chemical liquid passage, a decrease in the chemical liquid administration function of the needle tube 11 can be avoided. Further, the measurement accuracy of a temperature of the needle tube 11 at a position close to the inclined cutting edge surface P of the needle tube 11 is improved.

### Description of Reference Numerals

10: first unit
11: needle tube
12: needle base
120: winding portion
121: distal portion
122: proximal portion
14: thermocouple
16: chemical liquid tube
20: second unit
22: thermocouple connector
111: first needle tube
112: second needle tube
220: body (housing)
222: terminal
1110: insulating tube
1120: insulating tube
2220: insulating base body
2221: tip end recessed portion
2222: rear end recessed portion
C1: temperature measurement contact
C2: compensation contact
C4: reference temperature contact
P: inclined cutting edge surface
Q0: high-frequency channel
Q1: thermocouple channel
Q11: first thermocouple channel
Q12: second thermocouple channel
R: groove
S: local region (designated region)
T1: first connector
T2: second connector
W0: high-frequency wire
W1+, W1-: thermocouple wire
W2+, W2-: compensation wire

## Claims

1. A high-frequency electrode needle having a temperature measurement function, comprising:
a tubular needle tube having an inclined cutting edge surface formed at a needle tip;
a tubular needle base to which the needle tube is attached at a tip end portion;
a thermocouple composed of a pair of thermocouple wires electrically connected at a temperature measurement contact that is disposed at a tip end portion of the needle tube; and
a high-frequency wire disposed along an outside of the needle base and electrically connected to the needle tube to form a high-frequency contact,
wherein the temperature measurement contact is disposed in a state of being positioned at a rear side of the inclined cutting edge surface in a lumen of the needle tube.

2. The high-frequency electrode needle having a temperature measurement function according to claim 1,
wherein the temperature measurement contact is disposed in a state of being positioned at a front side of a rear end portion of the inclined cutting edge surface and at the rear side of the inclined cutting edge surface in the lumen of the needle tube.

3. The high-frequency electrode needle having a temperature measurement function according to claim 1,
wherein each of the pair of thermocouple wires is fixed to the needle base in a state of being interposed between an inner surface of the needle base and an outer surface of a chemical liquid tube that is inserted into inside of the needle base and liquid-tightly bonded, so that the temperature measurement contact is disposed in a state of being positioned in the lumen of the needle tube.

4. The high-frequency electrode needle having a temperature measurement function according to claim 1,
wherein a thermocouple channel is formed through a side wall of the needle tube in a longitudinal direction, and
each of the pair of thermocouple wires is disposed in an internal space of the thermocouple channel, and the temperature measurement contact is disposed in a state of being positioned in the internal space of the thermocouple channel as the lumen of the needle tube.

5. The high-frequency electrode needle having a temperature measurement function according to claim 4,
wherein the needle tube includes a first needle tube having the inclined cutting edge surface formed at a needle tip, and a second needle tube connected to a rear end portion of the first needle tube,
the first needle tube has a first thermocouple channel formed through a side wall of the first needle tube in the longitudinal direction,
the second needle tube has a high-frequency channel formed through a side wall of the second needle tube in the longitudinal direction, and a second thermocouple channel formed through the side wall of the second needle tube in the longitudinal direction and configured to communicate with the first thermocouple channel,
the high-frequency wire is inserted into the high-frequency channel from a rear end portion of the high-frequency wire, and is electrically connected to the rear end portion of the first needle tube to form a high-frequency contact, and
each of the pair of thermocouple wires is disposed in the internal space of the thermocouple channel which includes the first thermocouple channel and the second thermocouple channel communicating with each other, and the temperature measurement contact is disposed in a state of being positioned in an internal space of the first thermocouple channel as the lumen of the needle tube.

6. The high-frequency electrode needle having a temperature measurement function according to claim 1,
wherein the temperature measurement contact is formed by electrically connecting one of the pair of thermocouple wires to the needle tube serving as the other of the pair of thermocouple wires.

7. The high-frequency electrode needle having a temperature measurement function according to claim 1,
wherein an insulating tube is disposed in the lumen of the needle tube in a state of being positioned with respect to the needle tube, and
each of the pair of thermocouple wires is disposed in an internal space of the insulating tube, and the temperature measurement contact is disposed in a state of being positioned in the lumen of the needle tube and the internal space of the insulating tube.

8. The high-frequency electrode needle having a temperature measurement function according to claim 1,
wherein the needle tube is covered with an insulating member except for a designated region.

9. A high-frequency electrode needle having a temperature measurement function, comprising:
a tubular needle tube having an inclined cutting edge surface formed at a needle tip;
a tubular needle base to which the needle tube is attached at a tip end portion;
a thermocouple composed of a pair of thermocouple wires electrically connected at a temperature measurement contact that is disposed at a tip end portion of the needle tube; and
a high-frequency wire disposed along an outside of the needle base and electrically connected to the needle tube to form a high-frequency contact,
wherein in a state in which a gap between an outer surface of the needle tube and an inner surface of an insulating tube extends in a longitudinal direction of the needle tube, and in a state in which the outer surface of the needle tube and the inner surface of the insulating tube are bonded at a position except for the gap, at least a part of the outer surface of the needle tube is covered with the insulating tube, and
the pair of thermocouple wires are inserted into the gap from a rear end portion of the pair of thermocouple wires, and the temperature measurement contact is disposed in a state of being positioned at a rear side of the inclined cutting edge surface in an internal space of the insulating tube.
